# EUROPEAN PATENT APPLICATION

(11) **EP 2 684 535 A1**
(43) Date of publication of application: **15.01.2014**
(21) Application number: 12179704.7
(22) Date of filing: 08.08.2012
(51) Int. Cl.: A61C 1/00, C02F 1/78, A61L 2/20

(54) **Dental water disinfector**

(30) Priority: 12.07.2012 US 201213548128
(71) Applicant: Green, George, Coral Springs, FL 33067 (US); Union Dental Holdings Inc., Coral Springs, FL 33071 (US); Drinkable Air Inc., Lauderdale Lakes, FL 33311 (US)
(72) Inventor: Green, George, Coral Springs, FL Florida 33067 (US)
(74) Representative: Bates, Daniel Joseph

(57) **Abstract**

The present invention generally relates to water disinfection systems for use in dental operatory settings. In particular, a dental water disinfector is described, wherein the dental water disinfector is configured to purify water used during a dental procedure. In preferred embodiments of the present invention, the dental water disinfector is utilized to eliminate bacterial contaminants present in water supplies utilized by dental drills and irrigation equipment.

## Description

### FIELD OF THE INVENTION

The present invention generally relates to a water disinfectant system for use in dental operatory settings. In particular, a dental water disinfector is described, wherein the dental water disinfectant is configured to remove viable bacteria from the water used during dental procedures. In preferred embodiments of the present invention, the dental water disinfector is utilized to eliminate viable pathogenic bacteria present in water supplies utilized by dental drills and irrigation devices.

### BACKGROUND

Waterlines, reservoirs and other devices utilizing water in the dental operatory setting are subject to significant pollution by a variety of undesirable contaminants. Most notably, these include pathogenic bacterial contaminants that can be harmful and may be spread through the use of the contaminated devices.

This problem has been noted in numerous scholarly journals and other publications. It is well known that microbial contamination is common in dental waterlines. The lines are conducive to the growth of bacteria, including *Legionella* bacteria which is the cause of Legionnaires' disease.

Contamination of these waterlines can occur rapidly, with less than 5 days being required for total contamination of a waterline, even in newly installed waterlines. It has been argued that these waterlines and systems present in dental offices should be considered independent aquatic ecosystems in which opportunistic pathogens successfully colonize synthetic surfaces. In these ecosystems, pathogens easily colonize to potentially dangerous levels.

Currently, there are no appropriate systems, devices or methods for cleaning contaminants viable pathogenic bacteria from these waterlines and systems. Clearly, this is a dangerous situation where dental patients may be exposed to undesirable pathogens even during routine procedures.

Therefore, there is an overwhelming need in the art for a dental water purifier configured to eliminate or reduce bacterial contaminants from waterlines and water supplies. These and other features and advantages of the present invention will be explained and will become obvious to one skilled in the art through the summary of the invention that follows.

### SUMMARY OF THE INVENTION

Accordingly, it is an object of the present invention to provide a dental water disinfector configured to eliminate viable bacterial and other contaminants from waterlines and water supplies.

According to an embodiment of the present invention, a dental water disinfector includes: an ozone generator; a compressor operably connected to said ozone generator, wherein said compressor is configured to blend ozone from said ozone generator with air in a pressurized format; and a bubbler means configured to receive a pressurized air and ozone blend from said compressor in order to intersperse said air and ozone blend into a volume of water used in a dental water reservoir system.

According to an embodiment of the present invention, the dental water disinfector may include one or more solenoids configured to allow for the dental water system to move from a purification mode into an operative mode.

According to an embodiment of the present invention, the dental water disinfector may include a backflow valve configured to prevent water from flowing from an operative component of said dental water system into a container of said dental water system, wherein said container holds said volume of water used in said dental water system.

According to an embodiment of the present invention, the dental water disinfector may include a backflow valve configured to prevent high pressure air from flowing back into the ozone generator.

According to an embodiment of the present invention, the dental water disinfector may include a filter configured to prevent free oxygen from the air from being vented. In certain embodiments, the filter is a carbon filter.

According to an embodiment of the present invention, the dental water disinfector may include logic and control means configured to control operation of the dental water disinfector.

According to an embodiment of the present invention, the logic and control means is a printed circuit board.

According to an embodiment of the present invention, the logic and control means is configured to effect the change between a purification state and an operating state of said dental water system.

According to an embodiment of the present invention, a method for disinfect dental water systems includes the steps of: forcing ozone enriched air into a container of water used in a dental water system; and pressurizing contents of said container of water for use in said dental water system.

According to an embodiment of the present invention, the ozone is created via an ozone generator.

According to an embodiment of the present invention, the forcing of ozone enriched air into said container of water is accomplished through the use of a compressor and a bubbler means.

According to an embodiment of the present invention, the method further includes the step of receiving a disinfection cycle initiation event or activation of a microswitch.

According to an embodiment of the present invention, the disinfection cycle initiation event is caused by the depression of a button.

According to an embodiment of the present invention, the method further includes the step of generating an audible tone upon completion of a disinfection cycle.

According to an embodiment of the present invention, the method further includes the step of generating a visual cue upon completion of a disinfection cycle.

According to an embodiment of the present invention, the dental water system enters an inoperable state during said forcing step.

According to an embodiment of the present invention, the dental water system returns to an operable state after completion of said pressurizing step.

The foregoing summary of the present invention with the preferred embodiments should not be construed to limit the scope of the invention. It should be understood and obvious to one skilled in the art that the embodiments of the invention thus described may be further modified without departing from the spirit and scope of the invention.

### BRIEF DESCRIPTION OF THE DRAWINGS

FIG. 1 illustrates a schematic overview of a dental water disinfector, in accordance with an embodiment of the present invention; and

FIG. 2 illustrates a schematic of a dental water disinfector, in accordance with an embodiment of the present invention.

### DETAILED SPECIFICATION

The present invention generally relates to water disinfection systems for use in dental hygiene settings. In particular, a dental water disinfector is described, wherein the dental water disinfector is configured to purify water used to cool dental drills and rinse work areas of patients' mouths during a dental procedure. In preferred embodiments of the present invention, the dental water disinfector is utilized to eliminate bacteria and other contaminants present in water supplies utilized by dental drills and rinsing equipment.

Embodiments of the present invention are designed for use with dental operatory units whose water source is derived from a water reservoir and driven by compressed air to be delivered chairside to the patient during dental procedures. In certain embodiments, the dental water disinfector will fit common dental units in use today or units converted to air driven water reservoir systems

In a preferred embodiment of the present invention, the dental water disinfector is configured to disinfect water used during a dental procedure where the water is supplied from a pressurized bottle located on or within a few feet of the dental operatory. These components can collectively be referred to as the dental water system(s) or dental water supply system(s). In certain embodiments, the dental water disinfector uses ozone (03), a proven anti-microbial, to obtain and maintain the disinfection of water supplies utilized by the dental water systems.

Embodiments of the present invention are configured to automatically begin a cycle of forcing ozone enriched air into the bottle of water or other water supply once the water bottle or water supply is in place. In certain embodiments of the present invention, the cycle may only take a few seconds to a minute to complete. Longer or shorter cycles may be used depending on the nature of the dental water system, the aggressiveness of the cycle and other aspects of the dental water line disinfector. For instance, a quick cycle may be run that is of shorter duration, or a complete clean cycle may be run that is of a longer duration. One of ordinary skill in the art would appreciate that disinfection cycles, as they will be referenced to in this application, may be of numerous durations, and embodiments of the present invention are contemplated for use with disinfection cycles of any duration.

According to an embodiment of the present invention after the enriching process is complete an indicator will alert the dentist that the system is ready to proceed. At this point, water from the dental water system will be again pressurized and the water will be free to flow to the dental tools. In certain embodiments, a circuit may be included that allows the dentist to repurify the water if he/she desires due to the passage of time or for any other reason. The system may be manually engaged by the dentist at their convenience. For instance, a manual disinfection cycle may be selected through the depression of a button. This action activates the disinfection cycle (e.g., for less than one minute). At the end of this disinfection cycle, the indictor may once again notify the dentist that the system is ready to proceed.

According to an embodiment of the present invention, a dental water disinfector includes an ozone generator, a compressor and a bubbler means. Optionally, the system may be further comprised of one or more solenoids, one or more backflow valves, one or more filters and one or more control systems.

According to an embodiment of the present invention, the Ozone generator is configured to generate ozone that will be blended with air for the purpose of incorporating ozone into the dental water supply system. In certain embodiments, the ozone generator may be a commercial ozone generator device. One of ordinary skill in the art would appreciate that there are numerous types of ozone generators that may be utilized with embodiments of the present invention, and embodiments of the present invention are contemplated for use with any type of ozone generator.

According to an embodiment of the present invention, the compressor is configured to develop low pressure air, for instance 1-5 PSI, during the disinfection cycle that will blend with ozone and force that ozone into the water of the dental water supply system. One of ordinary skill in the art would appreciate that there are numerous types of compressors that may be utilized with embodiments of the present invention, and embodiments of the present invention are contemplated for use with any type of compressor.

According to an embodiment of the present invention, the bubbler means is configured to develop fine bubbles under or in the dental water supply system when supplied with pressurized air/ ozone blend. Pumping of ozone/air blended bubbles into the water of the dental water supply system works to provide an antimicrobial effect that kills and eliminates bacteria and other contaminants from the dental water supply systems. In a preferred embodiment of the present invention, the bubbler means may be a stone bubbler. One of ordinary skill in the art would appreciate that there are numerous types of bubbler means that may be utilized with embodiments of the present invention, and embodiments of the present invention are contemplated for use with any type of bubbler means.

According to an embodiment of the present invention, the dental water disinfector may include one or more solenoids. A first pneumatic solenoid may be utilized, the first pneumatic solenoid being normally open for the continuous pressurization of water such that flow is activated by the equipment inherent to the dental chair as it would without the dental water disinfector. When the dental water disinfector is switched to disinfection mode, the incoming air pressure will be shut off by this first pneumatic solenoid and re-established by reopening this first pneumatic solenoid at the end of the disinfection cycle.

According to an embodiment of the present invention, the second solenoid is may be a pneumatic solenoid normally closed to maintain pressure in the water bottle. The second pneumatic solenoid is activated to release pressure during the disinfection process. At the end of the disinfection process, the second pneumatic solenoid is closed allowing normal operations.

According to an embodiment of the present invention, the dental water disinfector may include one or more backflow valves. A first backflow valve may be utilized to prevent water from flowing backwards from the dental tools, chair or other component of the dental water supply system back to the water bottle.

According to an embodiment of the present invention, a second backflow valve may be utilized to prevent high pressure, for instance pressure around or greater than 40 PSI, air from flowing back to the ozone generator. Some ozone generator models have a backflow valve built in, thereby eliminating a need for replication.

According to an embodiment of the present invention, the dental water disinfector may further be comprised of a filter. The filter may be, for instance, a commercial filter device that is used to reduce the free oxygen from the air being vented during the disinfection cycle. Filters may include, but are not limited to, carbon filters. One of ordinary skill in the art would appreciate that there are numerous types of filters that could be utilized with embodiments of the present invention.

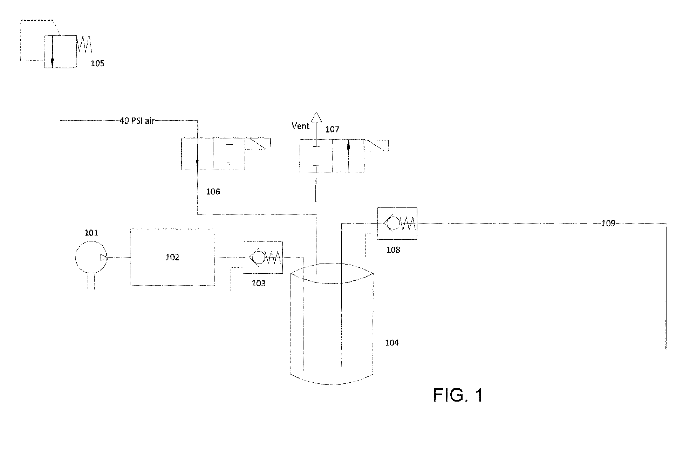

Turning to FIG. 1, a preferred embodiment of the present invention is shown. In this embodiment, an air compressor 101 is attached to an ozone generator 102 which runs through a first backflow valve 103 to the dental water system reservoir 104. An external air regulator 105 is attached to the dental water system reservoir 104 through a second pneumatic solenoid 106 and operably attached to a vent operated by a first pneumatic solenoid 107. A second backflow valve 108 connects the dental water system reservoir 104 to the other components 109 of the dental water system.

According to an embodiment of the present invention, the dental water disinfector may be comprised of one or more control and logic means. For instance, the dental water disinfector may include a control board comprised of a printed circuit board that controls the logic for the device. The control and logic means is configured to provide control operations with respect to both the automatic and manual disinfection cycles. The control and logic means may further be configured to allow for input and transmission of data with respect to the system, such as allowing the dentist or other operator to set times for disinfection cycles to run automatically and set cycle lengths, provide feedback as to when the last disinfection cycle was run, provide feedback as to whether disinfection is overdue or if the system is appropriately disinfected. One of ordinary skill in the art would appreciate that there are numerous input and transmission types that could be utilized with embodiments of the present invention, and embodiments of the present invention are contemplated for use with any type of input and transmission type. Further, one of ordinary skill in the art would appreciate that there are numerous types of control and logic means that may be utilized with embodiments of the present invention, and embodiments of the present invention are contemplated for use with any control and logic means.

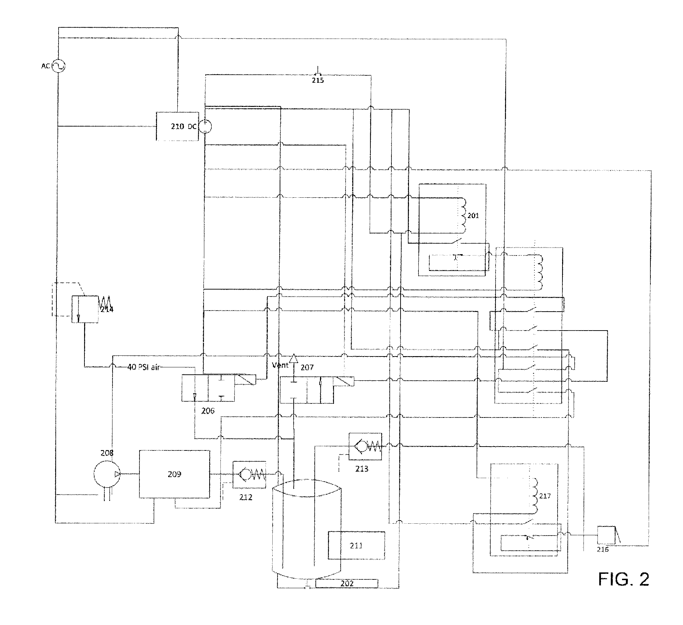

Turning now to FIG. 2, the control means may be configured with a micro-switch 202, which is activated by the insertion of the water reservoir bottle, to activate a time delay relay 201. This relay will close for a time X (e.g., less than one minute) which will run the disinfection cycle. This relay will activate relay circuits that closes solenoid 206 to stop line air pressure, opens solenoid 207 to vent pressure during the cycle, turns on the compressor 208 and the ozone generator 209. When this time relay circuit 201 is reopened, the solenoids 206, 207 will return to their default conditions, the compressor 208 and the ozone generator 209 will shut down. Additionally, a relay 217 activates a circuit controlling an indicator/ buzzer 216 for a few seconds the indication that the cycle is complete. A push button switch 215 is included to activate a disinfection cycle at any time. When this push button switch 215 is activated, the time delay relay 201 is activated just as above, which runs the same cycle as done when a new bottle is installed. Also shown in this FIG. 2 is a power supply 210, water reservoir 211, a first backflow valve 212, a second backflow valve 213 and an external air regulator 214.

While multiple embodiments are disclosed, still other embodiments of the present invention will become apparent to those skilled in the art from this detailed description. The invention is capable of myriad modifications in various obvious aspects, all without departing from the spirit and scope of the present invention. Accordingly, the drawings and descriptions are to be regarded as illustrative in nature and not restrictive.

## Claims

1. A dental water disinfector comprising:
an ozone generator;
a compressor operably connected to said ozone generator, wherein said compressor is configured to blend ozone from said ozone generator with air in a pressurized format; and
a bubbler means configured to receive a pressurized air and ozone blend from said compressor in order to intersperse said air and ozone blend into a volume of water used in a dental water system.

2. The dental water disinfector of claim 1, further comprising a one or more solenoids configured to allow for the dental water system to move from a disinfection mode into an operative mode.

3. The dental water disinfector of claim 1, further comprising a backflow valve configured to prevent water from flowing from an operative component of said dental water system into a container of said dental water system, wherein said container holds said volume of water used in said dental water system.

4. The dental water disinfector of claim 1, further comprising a backflow valve configured to prevent high pressure air from flowing back into the ozone generator.

5. The dental water disinfector of claim 1, further comprising a filter configured to prevent free oxygen from the air from being vented.

6. The dental water disinfector of claim 5, wherein said filter is a carbon filter.

7. The dental water disinfector of claim 1, further comprising a logic and control means configured to control operation of the dental water disinfector.

8. The dental water disinfector of claim 7, wherein said logic and control means comprises a printer circuit board; or wherein said logic and control means is configured effect the change between a disinfection state and an operating state of said dental water system.

9. A method for disinfecting dental water systems, the method comprising the steps of:
forcing ozone enriched air into a container of water used in a dental water system; and
pressurizing contents of said container of water for use in said dental water system.

10. The method of claim 9, wherein said ozone is created via an ozone generator; or wherein the forcing of ozone enriched air into said container of water is accomplished through the use of a compressor and a bubbler means.

11. The method of claim 9, further comprising the step of filtering free oxygen from air being vented via a filter.

12. The method of claim 11, wherein said filter is a carbon filter.

13. The method of claim 9, further comprising the step of receiving a disinfection cycle initiation event.

14. The method of claim 13, wherein said disinfection cycle initiation event is caused by the depression of a button.

15. The method of claim 9, further comprising the step of generating an audible tone upon completion of a disinfection cycle; or further comprising the step of generating a visual cue upon completion of a disinfection cycle; or wherein said dental water system enters an inoperable state during said forcing step; or wherein said dental water system returns to an operable state after completion of said pressurizing step.
